# EUROPÄISCHE PATENTSCHRIFT

(11) **EP 1 166 758 B1**
(45) Veröffentlichungstag und Bekanntmachung des Hinweises auf die Patenterteilung: **09.11.2005**
(21) Anmeldenummer: 01115548.8
(22) Anmeldetag: 28.06.2001
(51) Int. Cl.: A61K 7/13

(54) **Verfahren zum oxidativen Färben von menschlichen Haaren**
Method for oxidative dying of human hair
Méthode pour teinture oxidative des cheveux humains

(30) Priorität: 29.06.2000 DE 10031612
(43) Veröffentlichungstag der Anmeldung: 02.01.2002
(73) Patentinhaber: KPSS-Kao Professional Salon Services GmbH, 64297 Darmstadt (DE)
(72) Erfinder: Theis, Heinz, Dr., 64354 Reinheim (DE); Nöcker, Bernd, Dr., 64372 Ober-Ramstadt (DE); Wilz, Rüdiger, 64297 Darmstadt (DE)

(56) Entgegenhaltungen:
- EP-A- 0 962 218
- US-A- 3 975 515
- US-A- 5 053 051

## Beschreibung

Die vorliegende Erfindung betrifft ein neues Verfahren zum oxidativen Färben von menschlichen Kopfhaaren, das nicht nur haarschonender arbeitet als bisher bekannte und übliche Verfahren, sondern auch eine verbesserte Deckkraft und Farbintensität bzw. -stabilität bewirkt.

Die nach wie vor übliche Art der Haarfärbung ist die Färbung mit Oxidationsfarbstoffen, die unmittelbar vor der Anwendung mit Oxidationsmittel-Zusammensetzungen, insbesondere auf Basis von verdünntem Wasserstoffperoxid, vermischt und auf das Haar aufgetragen werden.

Der pH-Wert dieser gebrauchsfertigen Zusammensetzung liegt dabei in der Regel im alkalischen Bereich, insbesondere bei pH-Werten zwischen etwa 8 und etwa 11.

Durch diese alkalische Behandlung wird das Haar, insbesondere bei häufiger Wiederholung des Färbevorganges, in seiner Struktur geschädigt, vor allem, wenn es sich um bereits vorgeschädigtes Haar handelt. Dieses poröse Haar weist nach der Färbung auch eine schlechte Farbbeständigkeit auf.

Es wurde bereits vorgeschlagen, diese Nachteile der bisher üblichen Haarfärbung dadurch zu überwinden, daß, in Abkehr von der oben beschriebenen Praxis, anstelle der alkalisch eingestellten Zusammensetzungen aus Oxidationsfarbstoff und Oxidationsmittel eine analoge Zusammensetzung eingesetzt wird, deren pH-Wert im schwach sauren Bereich zwischen etwa 5,9 und 6,9 liegt.

Derartige Mittel, die z.B. in den DE-Osen Nr. 35 30 270, 36 28 397 und 36 28 398 beschrieben sind, haben sich in der Tat als wesentlich weniger haarschädigend als alkalische Produkte erwiesen.

In manchen Fällen, d.h., bei Einstellung spezieller Farbnuancen, läßt allerdings die Intensität und Stabilität der mit diesen verbesserten Zusammensetzungen erzielten Haarfärbungen etwas zu wünschen übrig.

Eine Verbesserung dieser bekannten Verfahren ist Gegenstand der DE 198 25 133 C1, das dort geoffenbarte Oxidationshaarfärbeverfahren besteht aus den folgenden Verfahrensschritten:

Aufbringen einer alkalisch eingestellten, mindestens eine Entwickler- und mindestens eine Kupplersubstanz sowie ein Oxidationsmittel enthaltenden Oxidationsfarbstoffmischung auf das Haar; anschließend, nach vorzugsweise etwa zehn- bis dreißigminütiger Einwirkung, ohne zwischenzeitliches Ausspülen Aufbringung einer mindestens eine Säure enthaltenden Zusammensetzung auf das Haar, und Spülen des Haares nach erfolgter Farbstoffeinwirkung.

US 3,975,515 beschreibt Verfahren zur Herabsetzung der AlkaliKonzentration in Alkali reagierenden kosmetischen Zusammensetzungen durch Einmischen von Ester und/oder Halogen enthaltende, Säure abspaltende Verbindungen kurz vor der Anwendung.

Die vorliegende Erfindung geht von der Aufgabenstellung aus, dieses Verfahren noch zu verbessern.

Diese Aufgabe wird dadurch gelöst, daß einer alkalisch eingestellten, mindestens eine Entwickler- und mindestens eine Kupplersubstanz sowie ein Oxidationsmittel enthaltenden Oxidationsfarbstoffmischung unmittelbar vor der Applikation auf das Haar eine Säure abspaltende Verbindung ausgewählt aus Gluconolacton, (Gluconsäure-5-lacton), Glucuronolacton, (Glucuronsäure-γ-lacton), Acetylsalicylsäure, Diethyloxalat, Galactonsäure-1,4-lacton und/oder 3,5-Dihydroxy-3-methyl-pentan-5-lacton, zugesetzt wird, die genügend Säure entwickelt, um den ursprünglichen pH-Wert der Farbmischung von etwa 8 bis 11, insbesondere 9 bis 10, innerhalb von etwa 10 bis 30 Minuten auf einen pH-Wert von etwa 5 bis 8, insbesondere etwa 6,5 bis 7,5, abzusenken, und die Mischung nach erfolgter Färbung, nach etwa 15 bis 40 Minuten Gesamteinwirkungszeit, aus dem Haar ausgewaschen wird.

Durch die Anwendung dieses Verfahrens wird eine kontinuierliche Absenkung des pH-Wertes der auf dem Haar befindlichen Farbmischung und damit eine schonende, gleichmäßige Haarfärbung mit einer ausgezeichneten Farbintensität erreicht.

Die Gesamtzeitspanne der Farbeinwirkung kann durch die Applikation von Wärme, vorzugsweise etwa 30 bis 50°C, beispielsweise etwa 40°C, um etwa ein Viertel bis zur Hälfte reduziert werden.

Die zum Einsatz gelangenden, alkalisch eingestellten Oxidationsfarbstoffmischungen, die mindestens je eine Entwickler- und je eine Kupplersubstanz sowie ein Oxidationsmittel enthalten, sind an sich bereits bekannt.

Zu diesen gebrauchsfertigen (d.h., das Peroxid enthaltenden) Mischungen, deren pH-Wert zwischen etwa 8 und 11, vorzugsweise 9 und 10, insbesondere bei etwa 9,5 liegt, wird hierzu auf den Stand der Technik, z.B. auf die Monographie von K. Schrader, "Grundlagen und Rezepturen der Kosmetika", 2. Aufl., S. 784-804 (1989), verwiesen; die dort beschriebenen Produkte sind im Rahmen des erfindungsgemäßen Verfahrens ebenso einsetzbar wie die aus dem umfangreichen Stand der Technik bekannten weiteren Entwickler- und Kupplersubstanzen sowie Nuanceure.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2-Hydroxyethylamino)-5-aminotoluol und 1-Amino-4-bis-(2'-hydroxyethyl)-aminobenzol bzw. deren wasserlöslichen Salze; beispielhafte Kupplersubstanzen sind Resorcin, 2-Methylresorcin, 4-Chlorresorcin, 2-Amino-4-chlorphenol, 4-(N-methyl)aminophenol, 2-Aminophenol, 3-Aminophenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodiphenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl) amino]benzol, α-Naphthol, 1,4-Diamino-2-chlor-benzol, 4,6-Dichlorresorcin, 1,3-Diaminotoluol, 4-Hydroxy-1,2-methylendioxybenzol, 1,6-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol und/oder 1-Methoxy-2-amino-4-(2'-hydroxyethylamino)benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in den erfindungsgemäß eingesetzten Farbmischungen kann jeweils etwa 0,25 bis etwa 5 Gew.-%, je nach gewünschter Färbung, betragen.

Als Öxidationsmittel werden vor allem verdünnte Wasserstoffperoxid-Lösungen, -Emulsionen oder -Gele eingesetzt, möglich, aber weniger üblich ist auch die Verwendung weiterer Peroxide wie Erdalkaliperoxiden, Harnstoffperoxid, Melaminperoxid, etc. in entsprechenden stöchiometrischen Mengen.

Die Oxidationsfarbstoffzusammensetzungen können als Lösungen, Cremes, Pasten, Gele, Aerosole, etc. Verwendung finden.

Die Gesamteinwirkungszeit auf dem Haar liegt vorzugsweise bei jeweils etwa zehn bis vierzig Minuten. Das erfindungsgemäße Verfahren eignet sich dabei sowohl zum ganzheitlichen, d.h. erstmaligen Färben der Haare als auch zum Nachfärben.
Als Säureabspalter eignen sich insbesondere Gluconolacton, (Gluconsäure-5-lacton), Glucuronolacton, (Glucuronsäure-γ-lacton), Acetylsalicylsäure, Diethyloxalat, Galactonsäure-1,4-lacton und/oder 3,5-Dihydroxy-3-methyl-pentan-5-lacton.

Die Art und Menge der zu einer freien Säure verseifbaren bzw. hydrolysierenden Verbindung hängt natürlich vom Ausgangs- und erwünschten End-pH-Wert der Färbemischung auf dem Haar und der Hydrolyse- bzw. Verseifungsgeschwindigkeit des Säureabspalters selbst ab.

Der pH-Wert der auf dem Haar befindlichen Farbmischung nach vollständiger Freigabe der Säure liegt dabei vorzugsweise zwischen etwa 6 und 8, insbesondere etwa 6,5 bis 7,5.

In den folgenden Beispielen wird die Erfindung illustriert.

### Beispiel 1

40g einer Färbezusammensetzung, die durch Vermischen von gleichen Gewichtsteilen einer 6%-igen Wasserstoffperoxidlösung und eines Oxidationsfarbstoffvorprodukts der folgenden Zusammensetzung erhalten wurde, und einen pH-Wert von 9,8 aufwies, wurden noch 40g Glucuronolactone zugesetzt und diese Mischung auf gebleichtes Menschenhaar aufgebracht.

### Trägermasse

| | |
|---|---|
| Cetylstearylalkohol | 11,00 (Gew.%) |
| Oleth-5 | 5,00 |
| Ölsäure | 2,50 |
| Stearinsäuremonoethanolamid | 2,50 |
| Cocosfettsäuremonoethanolamid | 2,50 |
| Natriumlaurylsulfat | 1,70 |
| Natriumsulfit | 1,00 |
| 1,2-Propandiol | 1,00 |
| Ascorbinsäure | 0,50 |
| Ammoniumchlorid | 0,50 |
| EDTA, Tetranatriumsalz | 0,20 |
| Parfum | 0,40 |
| Weizenproteinhydrolysat | 0,20 |
| Silica | 0,10 |

### Oxidationsfarbstoffmischung

| | |
|---|---|
| 2,5,6-Triamino-4-hydroxypyrimidinsulfat | 0,01 |
| 2,5-Diaminotoluolsulfat | 0,55 |
| 4-Chlorresorcin | 0,17 |
| Resorcin | 0,05 |
| 3-Aminophenol | 0,03 |
| Wasser | ad 100,00 |

Nach 15 Minuten war der pH-Wert der auf dem Haar befindlichen Farbmischung auf pH 7,1 gefallen und blieb dann konstant.

Nach weiterer 15-minütiger Einwirkung wurde gewaschen und getrocknet.

Es wurde eine starke, intensive, gleichmäßige Mittelblondfärbung erhalten. Das Haar wies einen lockeren Griff und eine volle Haarstruktur auf.

Weglassen des Glucuronolactons führte zu einem rauheren Haar mit deutlich verschlechterter Struktur.

Aufbringen eines 12,5%-igen wäßrigen Citronensäuregels nach 15-minütiger Einwirkung der alkalischen Färbemasse entsprechend dem in der DE 198 25 133 C2 beschriebenen Verfahren führte zwar ebenfalls zu einer pH-Absenkung auf 7,1; jedoch wurde eine weniger intensive, glänzende und vor allem gleichmäßige Färbung erhalten.

Auch Griff und Struktur des Haares waren weniger voll.

### Beispiel 2

In eine der Zusammensetzung nach Beispiel 1 entsprechende Trägermasse wurde die folgende Oxidationsfarbstoffmischung eingebracht:

| | |
|---|---|
| 2,5,6-Triamino-4-hydroxypyrimidinsulfat | 0,01 (Gew.-%) |
| 2,5-Diaminotoluolsulfat | 0,90 |
| 2-Amino-4-hydroxypyridin | 0,80 |
| 1-Naphthol | 0,17 |
| 3-Aminophenol | 0,10 |
| Resorcin | 0,03 |

Dieses Produkt wurde mit 6 %-iger H₂O₂-Lösung im Gewichtsverhältnis 1:1 vermischt (pH 9,5), zu 40g dieser Mischung 3g Acetylsalicylsäure zugesetzt, und auf Strähnen aus gebleichtem Menschenhaar aufgebracht.

Nach 15-minütiger Einwirkung wurde ein pH-Wert von 6,8 erreicht, der konstant blieb. Nach weiterer 15-minütiger Einwirkung wurde mit Wasser ausgewaschen, shampooniert und getrocknet.

Es wurde eine intensive, glänzende, gleichmäßige Mahagoni-Färbung erhalten, die auch nach fünf Haarwäschen noch stabil war.

Nach Anwendung ausschließlich einer alkalischen Zusammensetzung wies das Haar nicht nur eine weniger intensive Färbung, sondern auch einen rauhen Griff auf.

Die Einstellung des pH-Wertes auf etwa 6,8 nach 15-minütiger Einwirkung des alkalischen Färbemittels mit 15%-igem Weinsäuregel nach der DE 198 25 133 C2 und weiterer 15-minütiger Färbung ergab eine weniger intensive und gleichmäßige Farbe, auch Griff, Glätte und Struktur des Haares waren weniger gut.

## Patentansprüche

1. Verfahren zum oxidativen Färben von menschlichen Haaren, **dadurch gekennzeichnet, daß** eine alkalische eingestellte, mindestens eine Entwickler- und mindestens eine Kupplersubstanz sowie ein Oxidationsmittel enthaltende Oxidationsfarbstoffmischung unmittelbar vor dem Aufbringen auf das menschliche Haar mit einer eine Säure abspaltenden Verbindung ausgewahl aus Gluconolacton (Gluconsäure-5-lacton), Glucuronolacton, (Glucuronsäure-γ-lacton), Acetylsalicylsäure, Diethyloxalat, Galactonsäure-1,4-lacton und/oder 3,5-Dihydroxy-3-methyl-pantan-5-lacton vermischt und auf die Haare aufgebracht wird, wobei diese Verbindung genügend Säure entwickelt, um den ursprünglichen pH-Wert der Farbmischung von 9 bis 11 auf einen pH-Wert von 5 bis 8 abzusenken, und das Haar nach erfolgter Farbstoffeinwirkung gespült wird.

## Claims

1. Process for the oxidative dyeing of human hair, wherein an oxidation dyestuff mixture with an alkaline pH-value, comprising at least one developing, at least one coupling substance and an oxidizing agent is mixed, immediately prior to application onto the hair with, a compound releasing acids, selected from gluconolactone (gluconic acid 5-lactone), glucuronolactone (glucuronic acid γ-lactone), acetyl salicylic acid, diethyl oxalate, galactonic acid 1.4-lactone and/or 3.5-dihydroxy-3-methyl pentane-5-lactone, in an amount sufficient to reduce within a period of about ten to thirty minutes the original alkaline pH-value of the dyestuff mixture from 9 to 11 to 5 to 8, and rinsing the hair after the coloration is finished.

## Revendications

1. Procédé pour teindre par oxydation les cheveux humains, **caractérisé en ce qu'**un mélange de colorants par oxydation, rendu alcalin, contenant au moins une substance révélatrice et au moins une substance de couplage ainsi qu'un agent d'oxydation est mélangé directement avant de l'appliquer sur le cheveu humain, avec une substance libérant de l'acide, choisie parmi la gluconolactone (5-lactone de l'acide gluconique), la glucuronolactone (γ-lactone de l'acide glucuronique), l'acide acétylsalicylique, l'oxalate de diéthyle, la 1,4-lactone de l'acide galactonique et/ou la 3,5-dihydroxy-3-méthyl-pentane-5-lactone, et est appliqué sur les cheveux, cette substance libérant suffisamment d'acide pour abaisser le pH initial du mélange colorant de 9 à 11 à une valeur de pH de 5 à 8, et le cheveu est rincé après que l'action du colorant ait eu lieu.
